# EUROPÄISCHE PATENTANMELDUNG

(11) **EP 0 884 062 A1**
(43) Veröffentlichungstag der Anmeldung: **16.12.1998**
(21) Anmeldenummer: 97250183.7
(22) Anmeldetag: 13.06.1997
(51) Int. Cl.: A61M 16/10

(54) **Gerät zur Behandlung von virusbedingten Erkältungserscheinungen**

(71) Anmelder: Heising, Bernhard, 14059 Berlin (DE); Heising, Edelgard, 14059 Berlin (DE); Heising, Franziska, 14059 Berlin (DE); Heising, Cathrin, 14059 Berlin (DE)
(72) Erfinder: Heising, Bernhard, 14059 Berlin (DE); Heising, Edelgard, 14059 Berlin (DE); Heising, Franziska, 14059 Berlin (DE); Heising, Cathrin, 14059 Berlin (DE)
(74) Vertreter: Hengelhaupt, Jürgen, Dipl.-Ing.

(57) **Zusammenfassung**

Die Erfindung beschreibt ein Gerät zur Behandlung von virusbedingten Erkältungserscheinungen im Nasen-Rachen-Raum, insbesondere von Rhinitis, und ist bevorzugt anwendbar in der häuslichen Gesundheitspflege.
Eine gerätetechnische Realisierung der Erfindung besteht darin, daß mittels eines Heizelementes 6 erzeugte Heißluft 7 den Nasenhöhlen 3 über einen Inhalieraufsatz 2 zugeleitet wird.

## Beschreibung

Die Erfindung betrifft ein Gerät zur Behandlung von virusbedingten Erkältungserscheinungen im Nasen-Rachen-Raum, insbesondere von Rhinitis, und ist anwendbar zum Schutz und zur Wiederherstellung der Gesundheit. Bevorzugter Anwendungsbereich der Erfindung ist die häusliche Gesundheitspflege.

Es ist bekannt, virusbedingte Erkältungserscheinungen im Nasen-Rachen-Raum medikamentös zu behandeln. Nachteilig an einer derartigen Behandlung ist, daß die Medikamente relativ teuer sind, nur eine begrenzte Verwendungsdauer besitzen und unerwünschte Nebenwirkungen aufweisen können.

Beim Schnupfen (Rhinitis) handelt es sich um eine oberflächliche Entzündung der Nasenschleimhaut. Die akute Rhinitis wird vor allem durch Rhinoviren, aber auch durch andere Viren (Adenoviren, Parainfluenzaviren) hervorgerufen und verläuft mit oder ohne Fieber unter allgemeinem Krankheitsgefühl mit zunächst seröser, dann schleimig und eitriger Sekretion aus der Nase. Die Schwellung der Nasenschleimhaut behindert die Nasenatmung und beeinträchtigt das Riechvermögen.

Die Behandlung des Schnupfens beschränkt sich auf die Linderung der Symptome durch vaskonstriktorische Nasentropfen oder Inhalieren von mit Wirkstoffen (z.B. Kamille) angereicherter feuchtwarmer Luft. Nachteilig hierbei ist die relativ aufwendige Prozedur der Vorbereitung und die lange Zeitdauer der Durchführung des Inhaliervorganges.

Ebenfalls bekannte vorbeugende Impfungen gegen Schnupfen sind oftmals nicht wirksam, da es bekanntermaßen viele verschiedene Erreger gibt.

Der Erfindung liegt daher die Aufgabe zugrunde, ein Gerät zur Behandlung von virusbedingten Erkältungserscheinungen zu schaffen, welches einfach und preiswert herstellbar ist, jederzeit und kurzfristig anwendungsbereit zur Verfügung steht und die virusbedingten Erkältungserscheinungen unabhängig vom jeweiligen Erreger ohne unerwünschte Nebenwirkungen schnell heilt.

Eine einfach herzustellende und anzuwendende gerätetechnische Realisierung der Erfindung besteht darin, daß mittels eines Heizelementes erzeugte Heißluft den Nasenhöhlen definiert zugeleitet und in diese hineinbefördert wird, wobei die Zuleitung der Heißluft zu den Nasenhöhlen über einen Inhalieraufsatz erfolgen kann, welcher Nase und Mund des Gesichtes umschließt.

Ein besonderer Vorteil des erfindungsgemäßen Gerätes liegt darin, daß alle virusbedingten Erkältungserscheinungen im Nasen-Rachen-Raum, insbesondere der relativ häufig auftretende Schnupfen, unabhängig vom spezifischen Erreger behandelt werden können, indem den Nasenhöhlen bzw. dem Nasen-Rachen-Raum mehrmalig kurzzeitig trockene Heißluft zugeführt wird. Durch die Heißluft im Bereich von vorzugsweise 50°C - 60°C werden die temperaturempfindlichen Viren in ihrer Vermehrung und Ausbreitung gehindert bzw. abgetötet.

Die Erfindung soll nachstehend anhand von Ausführungsbeispielen näher erläutert werden.
Es zeigen:
- Figur 1: eine Prinzipdarstellung des Gerätes in Seitenansicht sowie der stilisiert dargestellten wesentlichen Bauteile;
- Figur 2: eine Vorderansicht des Inhalieraufsatzes

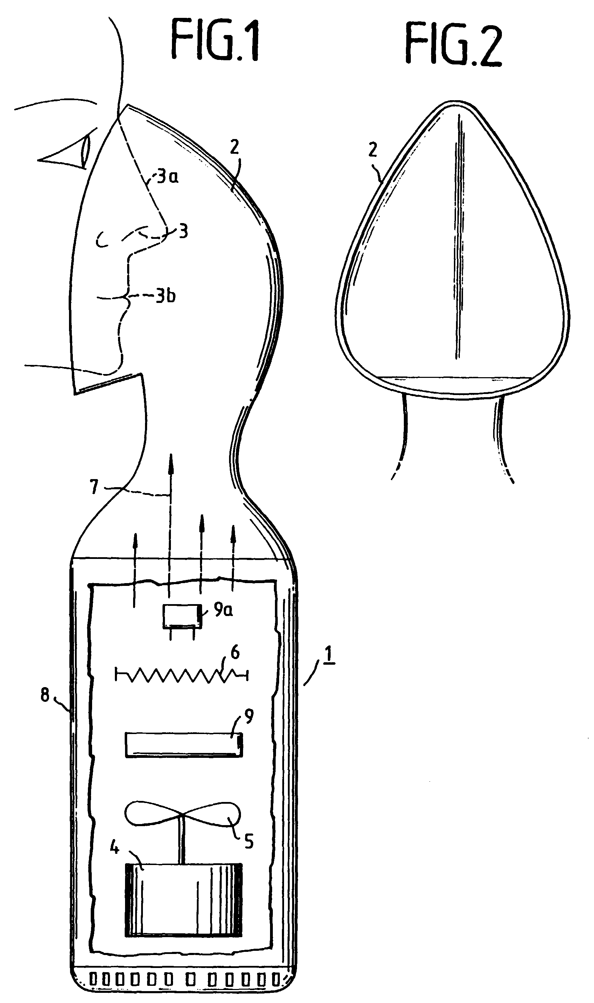

Wie aus Figur 1 zu ersehen ist, besteht das Gerät, welches zutreffend auch als Nasenfön bezeichnet werden kann, aus einem Gehäuse 8 mit einem Inhalieraufsatz 2. Der Inhalieraufsatz 2 ist im vorliegenden Ausführungsbeispiel so ausgebildet, daß er bei Aufsetzen auf das Gesicht Nase 3a und Mund 3b umschließt. Es ist aber auch möglich, den Inhalieraufsatz 2 so zu gestalten, daß lediglich eine Verbindung zu den Nasenhöhlen, beispielsweise durch Schläuche, Röhren oder Kanäle, besteht.

Im Handteil 1 des Gehäuses 8 ist ein Heizelement 6, ein Elektromotor 4, eine Regelschaltung 9, ein Temperatursensor 9a und ein Gebläse 5 angeordnet. Es ist ebenso möglich, den Temperatursensor 9a im Inhalieraufsatz 2 anzuordnen.
In der Figur 1 nicht dargestellt ist die Stromversorgung, welche sowohl mittels Batterien bzw. Akkumulatoren als auch über das Netz oder kombiniert erfolgen kann.

Bei Betrieb des Gerätes wird durch das Heizelement 6 die Heißluft 7 erzeugt, welche durch das vom Elektromotor 4 angetriebene Gebläse 5 in den Inhalieraufsatz 2 gedrückt und den Nasenhöhlen 3 und gegebenenfalls dem Mund 3b zugeleitet wird. Die Temperatur der Heißluft 7 wird von dem Temperatursensor 9a erfaßt und durch die Regelschaltung 9 auf einem vorwählbaren Wert konstant gehalten, indem die Regelschaltung auf das Heizelement 6 und/oder die Drehzahl des Elektromotors 4 einwirkt. Die Temperatur der Heißluft 7 wird im vorliegenden Ausführungsbeispiel mit einer Toleranz von ± 1°C konstant gehalten.
Eine einfache Realisierungsvariante des Gerätes besitzt eine Dreistufenschaltung, die es gestattet, die für die Nase verträgliche Temperatur einzustellen.

Die Hineinbeförderung der Heißluft 7 in die Nasenhöhlen 3 und den Mund 3b kann durch Einschnüffeln unterstützt werden.

Die in Figur 2 dargestellte Vorderansicht des Inhalieraufsatzes 2 zeigt den Raum, welcher Nase und Mund abdeckend umschließt sowie die Öffnung, aus welcher die Heißluft 7 in den Inhalieraufsatz 2 gelangt.

Eine bevorzugte Anwendungsvariante des Gerätes besteht darin, daß bei Beginn der Infektion alle 2 bis 3 Stunden 50°C - 60°C heiße Luft für ca. 10 bis 20 Sekunden der Nase und gegebenenfalls dem Mund zugeführt wird. Hierdurch werden die Rhinoviren unfähig, neue Zellen zu befallen. Durch die zugeführte warme Luft werden die Viren in ihren Lebensbedingungen, die bei Körpertemperatur optimal sind, gestört, wodurch es zu einem Abklingen der Virusproduktion kommt.

Eine beginnende Infektion verschwindet durch die Benutzung des Gerätes noch am gleichen Tag. Bei einem manifestierten Schnupfen ist eine Eigenbehandlung mit dem Gerät für eine Zeitdauer von ca. 2 Tagen erforderlich, danach ist der Schnupfen verschwunden.

Die kurze Dauer der jeweiligen Benutzung des Gerätes und die genaue Steuerung der Temperatur verhindern eine Austrocknung und Schädigung der Nasenschleimhaut und ein Fortschreiten der zerstörerischen Infektion. Hilfreich ist es auch, die Heißluft 7 nach dem Einleiten in die Nase 3a auch in den Mund 3b zu leiten, um die virusbefallenen Schleimhäute im Rachen-Raum ebenfalls zu erreichen.

Gegenstand der Erfindung ist nebem dem Behandlungsgerät auch die neue Verwendung von trockener Heißluft zur Behandlung von virusbedingten Erkältungserscheinungen im Nasen-Rachen-Raum gemäß den Ansprüchen 8-10.

Die Erfindung ist nicht auf die hier beschriebenen Ausführungsbeispiele beschränkt. Vielmehr ist es möglich, durch Kombination der beschriebenen Mittel und Merkmale weitere Ausführungsvarianten zu realisieren, ohne den Rahmen der Erfindung zu verlassen. Hierzu gehört auch die Verwendung eines herkömmlichen Gerätes zur Erzeugung von Heißluft, sofern es die dargestellten Kriterien erfüllt und insbesondere die Einstellung und Konstanthaltung der beschriebenen optimalen Temperaturen der Erfindung erlaubt, in Verbindung mit einem geeigneten Inhalieraufsatz. Somit liegen auch Nachrüstvarianten herkömmlicher Geräte im Rahmen der Erfindung.

### Bezugszeichenliste

- 1: Handteil
- 2: Inhalieraufsatz
- 3: Nasenhöhlen
- 3a: Nase
- 3b: Mund
- 4: Elektromotor
- 5: Gebläse
- 6: Heizelement
- 7: Heißluft
- 8: Gehäuse
- 9: Regelschaltung
- 9a: Temperatursensor

## Patentansprüche

1. Gerät zur Behandlung von virusbedingten Erkältungserscheinungen im Nasen-Rachen-Raum
dadurch gekennzeichnet,
daß mittels eines Heizelementes (6) erzeugte Heißluft (7) den Nasenhöhlen (3) zugeleitet und in diese hineinbefördert wird.

2. Gerät nach Anspruch 1,
dadurch gekennzeichnet,
daß die Zuleitung der Heißluft (7) zu den Nasenhöhlen (3) über einen Inhalieraufsatz (2) erfolgt.

3. Gerät nach Anspruch 1,
dadurch gekennzeichnet,
daß der Inhalieraufsatz (2) die Nase (3a) und den Mund (3b) des Gesichtes umschließt.

4. Gerät nach Anspruch 1,
dadurch gekennzeichnet,
daß das Heizelement (6) eine elektrisch betriebene Heizspirale ist.

5. Gerät nach Anspruch 1,
dadurch gekennzeichnet,
daß die Heißluft (7) mittels eines von einem Elektromotor (4) angetriebenen Gebläses (5) in die Nasenhöhlen (3) hineinbefördert wird.

6. Gerät nach einem der Ansprüche 1, 4 oder 5,
dadurch gekennzeichnet,
daß das Heizelement (6) und/oder das mittels Elektromotor (4) angetriebene Gebläse (5) durch eine Regelschaltung (9) geregelt werden.

7. Gerät nach Anspruch 1,
dadurch gekennzeichnet,
daß die Heißluft (7) auf eine Temperatur im Bereich von 40°C - 70°C einstellbar ist und eine vorzugsweise Einstellung auf 50-60°C aufweist.

8. Verwendung von trockener Heißluft zur Behandlung von virusbedingten Erkältungserscheinungen im Nasen-Rachen-Raum, insbesondere von Rhinitis.

9. Verwendung nach Anspruch 8,
dadurch gekennzeichnet,
daß die Heißluft auf eine Temperatur von 40-70°C, vorzugsweise von 50-60°C, eingestellt wird.

10. Verwendung nach Anspruch 8 oder 9,
dadurch gekennzeichnet,
daß bei einer beginnenden Infektion die heiße Luft alle 2 bis 3 Stunden der Nase und gegebenenfalls dem Rachenraum zugeleitet wird.

11. Gerät zur Behandlung von virusbedingten Erkältungserscheinungen im Nasen-Rachen-Raum, wobei mittelts eines Heizelementes erzeugte Heißluft einem Inhalieraufsatz zugeleitet wird,
dadurch gekennzeichnet,
daß die Heißluft (7) auf eine Temperatur im Bereich oberhalb von 52°C bis maximal 70°C einstellbar ist.
